# EUROPEAN PATENT APPLICATION

(11) **EP 4 183 291 A1**
(43) Date of publication of application: **24.05.2023**
(21) Application number: 22170447.1
(22) Date of filing: 28.04.2022
(51) Int. Cl.: A46B 15/00, A61C 17/22, A61B 5/00, A61C 19/04, G16H 15/00, G16H 80/00

(54) **PERMANENT TOOTH EMERGENCE ASSESSMENT**

(30) Priority: 22.11.2021 US 202163281902 P
(71) Applicant: Koninklijke Philips N.V., 5656 AG Eindhoven (NL)
(72) Inventor: HELAOUI, Rim, Eindhoven (NL); BRANDAO SILVA, Priscilla, Eindhoven (NL); RMAILE, Amir Hussein, Eindhoven (NL); KOOIJMAN, Gerben, Eindhoven (NL); JOHNSON, Mark Thomas, Eindhoven (NL)
(74) Representative: Philips Intellectual Property & Standards

(57) **Abstract**

A processing arrangement and method for providing guidance in relation to permanent tooth emergence of an infant. Embodiments propose to process an obtained indicator of tooth mobility of a primary (infant) tooth with a pre-determined algorithm or decision tool, and generate a prompt relating to possible adult tooth emergence dependent upon an outcome of the algorithm or decision tool. The prompt may be a recommendation to check for adult tooth emergence at a site of the relevant tooth. The prompt may be a prediction or estimate of a permanent tooth emergence status.

## Description

### FIELD OF THE INVENTION

The present invention relates to a device and method for providing user-assistance in relation to permanent tooth emergence of an infant, for example using sensing data obtained during use of an oral care device.

### BACKGROUND OF THE INVENTION

In most cases, a primary tooth (baby tooth) will fall out from an infant's mouth by natural processes and is soon replaced by a permanent (adult) tooth. However, in some cases, the shedding of a baby tooth and emergence of the permanent tooth can become desynchronized. For example, in some cases, permanent teeth emerge as a second row before the baby teeth fall out. In some other cases, a baby tooth will fall out before the permanent tooth is ready. Hence, in some cases, it may be required to manually extract a tooth to make room for the permanent tooth. Identifying whether or not extraction is needed, and identifying an optimal timing for such extraction is not straightforward, especially for inexperienced parents.

Tooth mobility can provide some guide to parents as to when a tooth may be ready for extraction. However, normal (physiological) tooth mobility e.g. due to chewing forces, varies between different teeth and ranges for example from 40 µm (molars) to 120 µm (incisors). Thus, a parent could mistake normal tooth mobility as in indicator that an infant tooth should be extracted.

Extracting a mobile baby tooth before it is ready can cause bleeding and pain. It can also risk infection. It may also lead to complications and requirement for remedial action, e.g. insertion of spacers if the permanent tooth is not likely to emerge for some time. Further to this, neighboring teeth may shift into the empty space created by the extracted tooth, causing crowding or spacing problems for permanent teeth. This is a known cause for malocclusion of permanent teeth. It has been reported that ~ 20- 65% children have had at least one premature extraction of a tooth. It has been reported that the premature loss or extraction of primary teeth can result in clinically relevant loss of space and arch length reduction.

The notwithstanding, waiting too long before performing tooth extraction can cause unnecessary discomfort to a child. It also increases the risk of infection, and can interfere with the growth of the corresponding permanent tooth.

### SUMMARY OF THE INVENTION

The invention is defined by the claims.

According to examples in accordance with an aspect of the invention, there is provided a processing arrangement for an oral care device, comprising: an input/output; and one or more processors.

The one or more processors are adapted to: obtain an indication of a degree of tooth mobility of at least one infant tooth in the mouth of a user of the oral care device; assess the degree of tooth mobility according to at least one pre-defined criterion for the tooth mobility; and responsive to the degree of tooth mobility meeting the at least one pre-defined criterion, either
generating an output signal at the input/output for causing a user interface element to generate a prompt recommending that a user manually check for emergence of a permanent tooth at the site of the at least one infant tooth; or
generating a control signal at the input/output for controlling acquisition of an indicator of permanent tooth emergence status based on sensing or image data for the at least one infant tooth.

Thus, embodiments propose performing automated decision support, whereby a tooth mobility level of a primary (infant) tooth is algorithmically processed to determine whether a permanent tooth may be emerging, and to provide thereby a prompt to a parent to manually check for emergence or to automatically check for emergence. In some embodiments, the processing arrangement may also generate a recommendation relating to whether or not to extract (or seek a professional opinion in relation to this), and/or at what future time extraction might be need to be performed or planned for. In some embodiments, sensing data acquired using the oral care device is utilized in one or more of: deriving the level of tooth mobility of a given tooth, determining/predicting an adult tooth emergence status, and generating a tooth extraction recommendation.

For example, in some embodiments, the processing arrangement may be further adapted to receive at the input/output sensing data related to an oral cleaning action performed using the device during an oral cleaning session. The degree of tooth mobility may be determined based on the sensing data. An alternative source of the degree of tooth mobility could be a user input, for example provided by a parent who manually checks the mobility level. Using the sensor data improves convenience for the user.

With regards to the sensing or image data, this may be acquired using a sensing element or an imaging element comprised by the oral care device, e.g. a camera integrated in a tip of the oral care device. Alternatively, the sensing or image data might be pre-acquired sensing or image data which is stored in a datastore and retrieved for this process.

In at least one set of embodiments, the processing arrangement is adapted to receive at the input/output a sensing signal indicative of a loading force applied to, or motion of, at least one cleaning element of the oral care device, wherein the signal spans a time period, the time period corresponding to a period of mechanical engagement of the at least one cleaning element with at least one tooth during an oral cleaning session, wherein the tooth is the at least one infant or primary tooth previously referred to. The processing arrangement may be adapted in this case to determine the measure indicative of a degree of tooth mobility for at least one tooth based on processing of the sensing signal.

The sensing signal provides the previously mentioned sensing data.

Alternatively, the processing arrangement may receive an indication of a degree of tooth mobility from a different source, e.g. manually input based on measurement with an auxiliary device.

In some examples, the processing arrangement may be adapted to process a waveform of the signal to extract one or more characteristics of the signal waveform for the time period, and to determine the measure indicative of a degree of tooth mobility in dependence upon the extracted one or more signal waveform characteristics.

The one or more signal characteristics may include one or more characteristics of a frequency spectrum of the signal, for example an amplitude of a dominant peak or frequency component (i.e. main harmonic component) of the frequency spectrum, or a bandwidth associated with a dominant peak in the frequency spectrum.

In some embodiments, further to providing the prompt to the user, the processing arrangement may be adapted to obtain an indicator of a permanent tooth emergence status at the site of the at least one infant tooth. This could be based for instance on user input and/or based on obtained sensing data.

In some embodiments, the indicator of a permanent tooth emergence status may comprise a binary parameter indicative of emergence or non-emergence.

In some embodiments, the indicator of a permanent tooth emergence status may comprise a graded score indicative of a level of emergence.

In some embodiments, an indicator of a permanent tooth emergence status may be obtained by processing the obtained degree of tooth mobility with a pre-determined tooth emergence algorithm.

In some embodiments, an indicator of a permanent tooth emergence status may be obtained based on image data of the site of the at least one infant tooth, received at the input/output from an image capture device, and based on processing of the image data with a predetermined image analysis algorithm.

In some embodiments, an indicator of a permanent tooth emergence status may be obtained based on receiving at the input/output a user input signal from a user interface element indicative of the permanent tooth emergence status.

In some embodiments, an indicator of a permanent tooth emergence status may be obtained by processing an obtained sensing signal with a pre-determined tooth emergence algorithm. Options relating to receipt of a sensing signal have been discussed above.

In some embodiments, the permanent tooth emergence status may be derived based on historical values of the tooth mobility, or a trend in these values. In particular, in some embodiments, the one or more processors may be adapted to: access a datastore recording historical tooth mobility data for the at least one tooth of the user; determine a longitudinal trend in the degree of tooth mobility for at least one tooth of the user based on the historical tooth mobility data and the determined degree of tooth mobility; and wherein the indicator of a permanent tooth emergence status is determined in dependence on the longitudinal trend.

Further to generating the prompt, in some embodiments, the processing arrangement may generate an infant tooth extraction recommendation. This may be generated for example based on a combination of the degree of tooth mobility and the indicator of permanent tooth emergence status. The processing arrangement may generate a data output indicative of the recommendation. This may be coupled to the input/output for communicating to a user interface element in some examples. It may be coupled to the input/output for communicating to a datastore in other examples. It other examples it may be used as a trigger signal to trigger a response action, for example

In some embodiments, the infant tooth extraction recommendation may comprise a binary recommendation indicating whether or not the at least one tooth should be extracted. In some embodiments, the infant tooth extraction recommendation may comprise recommended minimum time to wait before extracting the tooth.

In some embodiments, the one or more processors may be further adapted to obtain an identification of the at least one tooth, and/or an indication of a location of the at least one tooth in the mouth.

The identification of the tooth or location indication may in some embodiments be obtained based on a positioning signal for the at least one cleaning element received from a positioning means. The identification of the tooth or location indication may in some embodiments be obtained based on a user input.

In some embodiments, the prompt for recommending that a user manually checks for emergence of a permanent tooth may include an indication of a location of the at least one tooth.

In some embodiments, the tooth extraction recommendation is further based on a demographic classification of the user.

In some embodiments, the processing arrangement may be further adapted to receive from a user interface an input indicative of a subjective discomfort level of the user, and wherein determining the tooth extraction recommendation is further based thereon.

In some embodiments, and by way of example, the recommendation may be a recommendation to extract the tooth in the case that both: the permanent tooth emergence status is positive; and the mobility metric exceeds a predetermined extraction threshold.

In some embodiments, and by way of example, the at least one pre-defined criterion for the degree of tooth mobility may be a pre-defined threshold for the degree of tooth mobility.

A further aspect of the invention provides an oral care device which comprises the processing arrangement discussed above. The processing arrangement may comprise: a support body; a plurality of cleaning elements for engagement with oral surfaces, the cleaning elements protruding from a surface of the support body; and a processing arrangement in accordance with any embodiment described in this disclosure.

The device in some embodiments may further comprise a sensor means adapted to sense a measure indicative of a loading force or pressure applied to, or motion of, at least one of the cleaning elements.

By way of example, the oral care device may be a powered toothbrush, or may be a brushing mouthpiece device.

Another aspect of the invention provides a system comprising an oral care device as described above, and further comprising a user interface.

A further aspect of the invention provides a computer implemented method comprising:
obtaining an indication of a degree of tooth mobility of at least one infant tooth in the mouth of a user of an oral care device;
assessing the degree of tooth mobility with at least one pre-defined criterion for the mobility; and
responsive to the degree of tooth mobility meeting the at least one pre-defined criterion, either
   generating an output signal for causing a user interface element to generate a prompt recommending that a user manually check for emergence of a permanent tooth at the site of the at least one infant tooth; or
   generating a control signal for controlling acquisition of an indicator of permanent tooth emergence status based on sensing or image data for the at least one infant tooth.

Another aspect of the invention provides a computer program product comprising code means configured, when run on a processor, to cause the processor to perform the method recited above, or a method in accordance with any embodiment described in this disclosure.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows an example processing arrangement according to one or more embodiments;
Fig. 2 further illustrates an example processing arrangement according to one or more embodiments;
Fig. 3 illustrates an example process for generating a recommendation in relation to tooth extraction;
Fig. 4 illustrates an example processing arrangement in accordance with one or more embodiments;
Fig. 5 illustrates an example oral care device in accordance with one or more embodiments;
Figs. 6-7 illustrate a sensing principle for sensing force on, or motion of, cleaning elements of an oral care device;
Fig. 8 illustrates an example sensing signal waveform;
Fig. 9(a) illustrates an example frequency spectrum of a segment of a sensing signal waveform, and Fig. 9(b) illustrates computation of an example bandwidth of a dominant peak of a frequency spectrum; and
Fig. 10 illustrates an example processing flow according to one or more embodiments.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a processing arrangement and method for providing guidance in relation to permanent tooth emergence of an infant. Embodiments propose to process an obtained indicator of tooth mobility of a primary (infant) tooth with a pre-determined algorithm or decision tool, and generate a prompt relating to possible adult tooth emergence dependent upon an outcome of the algorithm or decision tool. The prompt may be a recommendation to check for adult tooth emergence at a site of the relevant tooth. The prompt may be a prediction or estimate of a permanent tooth emergence status.

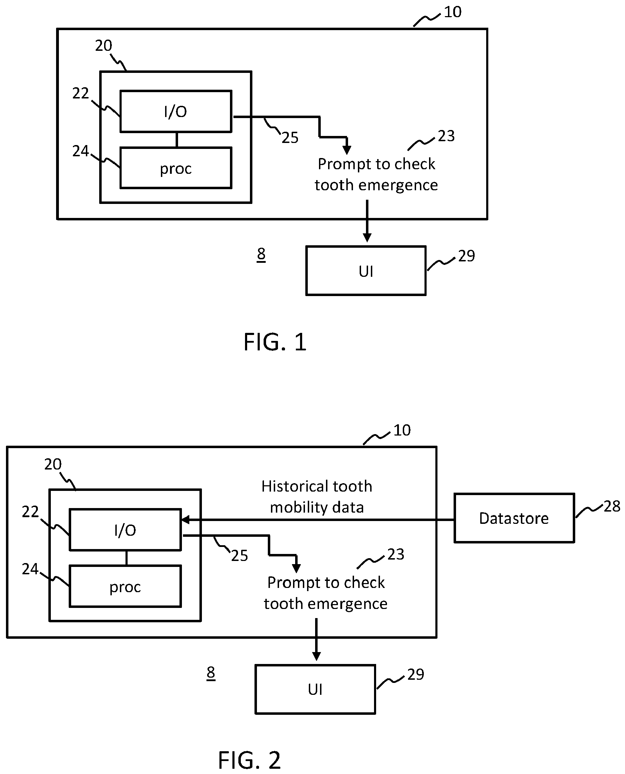

Fig. 1 illustrates an example processing arrangement 20 in accordance with one or more embodiments.

The processing arrangement 20 comprises an input/output 22; and one or more processors 24. The processing arrangement is for use in an oral care device 10, such as a powered toothbrush or brushing mouthpiece device.

The processing arrangement 20 is adapted to obtain an indication of a degree of tooth mobility of at least one infant tooth in the mouth of a user of the oral care device, and assess the degree of tooth mobility with at least one pre-defined criterion for the tooth mobility. Responsive to the degree of tooth mobility meeting the at least one pre-defined criterion, the processing arrangement is adapted to generate an output signal 25 at the input/output for causing a user interface 29 element to generate a prompt 23 recommending that a user manually check for emergence of a permanent tooth at the site of the at least one infant tooth.

Another aspect of the invention provides the oral care device 10 comprising the processing arrangement.

Another aspect of the invention provides a system 8 comprising the oral care device 10 (or at least comprising the processing arrangement 20) and further comprising a user interface 29.

In general, an (inexperienced) parent will not know when the emergence of a permanent tooth will occur, and generally will not systematically check for such an emergence. Furthermore, there is usually no system in place for the regular assessment of permanent tooth emergence in a clinical context.

As a consequence, it is proposed by embodiments of the present invention to provide a decision support flow implemented by a processing arrangement 20, in which presence of a certain degree or level of tooth mobility, or for instance a particular increase of a tooth mobility, is used as a basis for triggering a request to investigate for the emergence of a permanent tooth. For example, the at least one criterion mentioned above could be a minimum threshold for the tooth mobility. There may also be a second criterion for the tooth mobility, such as a second threshold, which is used as a basis to trigger a recommendation to actually extract the tooth. The first mobility threshold which triggers the request to investigate the emergence of the permanent tooth will in general be lower than the second threshold associated with the decision to extract the tooth.

For these purposes, it will be of considerable advantage if the system can further provide an indication of the location of the mobile tooth within the mouth, or an identification of the tooth which allows the location to be found, in order to guide the users (the parents) to the part of the mouth they need to investigate. In cases where the tooth mobility metric is based on sensing data acquired during an oral cleaning session, this could be facilitated for example by a positioning means integrated in the oral care device 10 such as an inertial measurement unit whose output can be used to determine an orientation of the device during acquisition of sensing data which is used to generate the relevant mobility metric for a tooth. Orientation can be mapped to location in the mouth at which a head of the device is directed, and thus which tooth is being cleaned. By way of one further example, a position indication can also be derived directly from the output of an inertial measurement unit, for instance based on a transfer function which has been pre-determined. The transfer function can be a machine learning algorithm in some examples.

The prompt generated at the user interface 29 may include an indication of a location of the at least one tooth.

The processing arrangement may be adapted to perform the above-recited steps for assessing tooth mobility and conditionally providing the recommendation for a plurality of different teeth in the infant's mouth. In most preferred cases, it may perform these steps for all teeth in the mouth. Thus, identifying a location of each tooth which is assessed in this way allows for the user to understand which tooth each recommendation corresponds to.

As will be discussed in more detail below, in some embodiments, the processing arrangement may be adapted to determine the degree or level of tooth mobility. This may be based on sensing data related to an oral cleaning action performed using the device during an oral cleaning session. For example, it may be based on a sensing signal indicative of a loading force or pressure applied to, or motion of, at least one cleaning element of the oral care device, or a support body 12 to which the cleaning element is attached, and where the signal corresponds to a period of mechanical engagement of the at least one cleaning element with at least one tooth during an oral cleaning session, wherein the tooth is the at least one infant/primary tooth. For example, load variations on the support body or the cleaning element(s) may be sensed.

In some embodiments, the processing arrangement may be further adapted to determine a permanent tooth emergence status.

Detection can be based on using the same sensing data as used to determine the degree of tooth mobility. For example, data may be utilized from a force or pressure sensing means integrated in a support body of the oral care device, which support body carries cleaning elements. Sensing data may be acquired during an oral cleaning session related to force or pressure exerted upon the cleaning elements when they are engaging teeth and surrounding gums. As a new adult tooth emerges, the firmness of the gum adjacent to the corresponding infant tooth will increase, and this can be detected as changes in the reaction force exerted upon cleaning elements when they touch the relevant area of the gum. Thus, the emergence of the tooth could be detected based on a trend over multiple cleaning sessions of the force sensor measurements for the corresponding area of the gum. In other words, the processing arrangement may compare trends of pressure changes in the gum surface adjacent to mobile baby teeth, where the locations of mobile baby teeth can be detected using the automated mobility detection mentioned above.

In some examples, the permanent tooth emergence status may be obtained based on the tooth mobility measurements. For example, the indicator of a permanent tooth emergence status may be obtained based on processing the obtained degree of tooth mobility with a pre-determined tooth emergence algorithm. In some examples, a trend in the tooth mobility measurements might be used to determine permanent tooth emergence status.

For example, with reference to Fig. 2, the processing arrangement 20 may be adapted to access a datastore 28 recording historical tooth mobility data for the at least one tooth of the user. It may store said data for multiple different teeth, and where the datastore is queried in accordance with a tooth of interest. The processing arrangement, using data from the datastore 28, may determine a longitudinal trend in the degree of tooth mobility for at least one tooth of the user based on the historical tooth mobility data, and optionally also based on the determined degree of tooth mobility from the current cleaning session. The indicator of a permanent tooth emergence status is determined in dependence on the longitudinal trend.

It is noted, with reference to Fig. 2, that in accordance with any embodiment of the present invention, the processing arrangement may be adapted to, for each cleaning session of the oral care device, store the obtained degree of tooth mobility for each tooth in the datastore 28, to thereby build up over time the dataset of historical tooth mobility data. One aspect of the invention may provide a system 8 which includes the processing arrangement 20 and the datastore 28 storing historical tooth mobility data. Another aspect may provide a system 8 comprising an oral care device which contains the processing arrangement 20, in combination with the aforementioned datastore 28, and optionally also comprising a user interface.

In some examples, the indicator of permanent tooth emergence status may be obtained from acquired image data of the site of the relevant infant tooth. Determination of the permanent tooth emergence status may be based on comparing intra-oral images or pictures taken daily for differences regarding tooth like features (i.e. the appearance of an extra tooth surface adjacent to an existing tooth). For this purpose, an image capture device (such as a camera) may be integrated in the oral care device, for example in a support body from which cleaning elements protrude. The image data may be processed with one or more predetermined image analysis algorithms for detecting image features characteristic of permanent tooth emergence.

In other examples, the determination may be based simply on a user input (from the child's parent or the child themselves) via the user interface 29.

Inputs from other sensing sources such as x-ray sensors or radar sensors or any other sensing modality may additionally or alternatively be used to detect emergence of tooth.

With regards to the content of the indicator of a permanent tooth emergence status, there are different options.

The indicator of a permanent tooth emergence status may comprise in some cases a binary parameter indicative of emergence or non-emergence of a permanent tooth at a particular tooth site. In some examples, the indicator may comprise a graded score indicative of a level of emergence.

In some embodiments, recommendation information may be provided aiming at optimal synchronization as to when to extract a given tooth. In other words, the processing arrangement may generate an infant tooth extraction recommendation. This may signal the optimal intervention moment for the mobile tooth.

In particular, in some examples, the processing arrangement may generate an infant tooth extraction recommendation for a given tooth in dependence upon the obtained degree of tooth mobility for the tooth and upon the indicator of permanent tooth emergence status. For example these two parameters may be fed as inputs to a tooth extraction algorithm which provides as an output a recommendation.

The processing arrangement 20 may generate a data output indicative of the recommendation and couple the data output to the input/output 22 for communicating to a user interface element.

With regards to the specific content of the infant tooth extraction recommendation, there are different options. In some examples, it may comprise a binary recommendation indicating whether or not the at least one tooth should be extracted, or at least whether or not extraction needs to be considered at the present time. In some examples, the infant tooth extraction recommendation may comprises a recommended minimum time to wait before extracting the tooth, or at least before seeking a professional opinion regarding extraction.

In some examples, determining the tooth extraction recommendation may be further based on a demographic classification of the user, for example their age, gender etc.

In some examples, the processing arrangement may prompt at the user interface 29 for input of a subjective discomfort level of the child user. Determining the tooth extraction recommendation may be further based on this subjective discomfort level.

In some examples, the recommendation may be a recommendation to extract the tooth in the case at least that both: the permanent tooth emergence status is positive; and the mobility metric exceeds a predetermined extraction threshold.

To provide the output recommendation, as well as other optional processing and decision support discussed in more detail hereafter, a Decision Support System or engine (DSS) can be utilized.

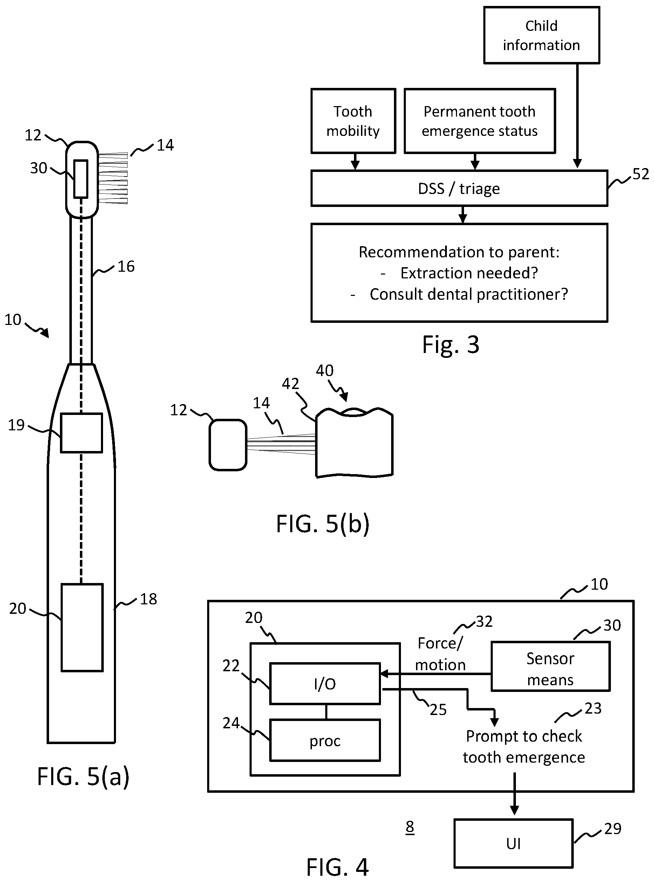

As illustrated in Fig. 3, the DSS 52 make take into account the obtained degree of tooth mobility, as well as the obtained indication of permanent tooth emergence status.

Optionally, the DSS 52 may additionally use child-specific information such as age, gender, ethnicity, other demographic information, and/or medical history information related to the child, or information about previous extractions or tooth release timings. This information may at least in part be input by a user via the user interface 29.

In some examples, the DSS 52 can additionally make use of a database containing information related to natural tooth release timing for a population of children.

A DSS is a functional entity, rather than a structural entity. It comprises one or more algorithms for example. It comprises application of a decision tree for example.

The DSS 52 processes the various inputs to provide an output recommendation to a parent of the child. This could include for example a recommendation to seek advice from a dental practitioner. It could include a recommendation related to a likely need for extraction. It could include a recommended timing for tooth extraction.

In some examples, the processing arrangement is further adapted to prompt the user to input to the user interface feedback regarding a previous tooth extraction, in particular related to how a tooth extraction went (e.g. bleeding) to improve future recommendations for the same child.

As mentioned above, in some embodiments the degree of tooth mobility can be derived automatically based on sensing data acquired during a cleaning session. This feature will now be discussed in detail, with reference to Figs. 4-9.

Fig. 4 illustrates an example processing arrangement 20 in accordance with this set of embodiments.

Fig. 5 illustrates an example oral care device 10 integrating a sensing means 30 which may be used to acquire the sensing data in accordance with this set of embodiments. The processing arrangement 20 of Fig. 4 may be integrated in the oral care device 10. Although an oral care device in the form of a toothbrush is shown in Fig. 5, this is exemplary only and a different type of oral care device may be used such as for example a brushing mouthpiece device, an oral irrigator, or a powered flossing device. The block diagram structure of Fig. 4 is applicable to an oral care device of any particular type, not just to a toothbrush.

With reference to Fig. 4 and 5, the processing arrangement 20 in this set of embodiments may be adapted to receive at the input/output 22 a sensing signal 32 indicative of a loading force or pressure applied to, or motion of, at least one cleaning element 14 of the oral care device, or the support body of the device 12 to which the cleaning elements are attached. The signal spans a time period, the time period corresponding to a period of mechanical engagement of the at least one cleaning element with at least one tooth 40 during an oral cleaning session. The tooth 40 is the at least one infant tooth previously discussed.

The processing arrangement 20 is adapted to determine the measure indicative of a degree of tooth mobility for at least one tooth based on processing of the sensing signal.

As mentioned, Fig. 5(a) shows an example oral care device in the form of a powered toothbrush. Fig. 5(b) illustrates the mechanical engagement of the cleaning elements 14 on a surface 42 of an example tooth 40.

The exemplary toothbrush device 10 has with a brush head 16 coupled to a main body 18. The main body forms a handle for the device. The support body 12 is provided by a platen of the brush head. The cleaning elements 14 may comprise bristles. The bristles may be arranged into tufts. The reference to at least one cleaning element above could be reference to at least one tuft of bristles in this set of embodiments.

Although bristles are mentioned as an example, the skilled person will be aware that other varieties of cleaning element are also possible, such as elastomeric cleaning elements (thicker than a typical individual bristle), or cleaning structures which protrude from the support body having extended shapes, but which are also designed to engage with oral surfaces to provide a mechanical cleaning action.

The oral care device 10 includes a sensor means 30 adapted to generate a sensing signal 32 indicative of a loading force applied to, or motion of, at least one cleaning element 14 of the oral care device, or the support body of the device 12 to which the cleaning elements are attached. The sensor means may be integrated in the support body 12 in some examples. In other examples, it could be located elsewhere, e.g. in the main body 18, arranged to sense pivotal forces between the brush head 16 and the main body 18.

With further reference to Fig. 5, preferably the oral care device includes an actuation means 19 for driving an oscillatory motion of the at least one cleaning element 14.

This induces oscillatory motion pattern of the at least one cleaning element exhibits a frequency spectrum. The device may record in a memory of the processing arrangement 20 (not shown) one or more reference frequency spectra which correspond to frequency spectra of the driven oscillatory motion pattern in certain reference or calibration conditions, such as in the presence of a certain reference loading force and to a certain tooth with a reference level of mobility (e.g. zero mobility). The reference condition could be any desired condition, e.g. in the absence of externally applied loading forces to the at least one cleaning element, and/or for a tooth with non-zero mobility. There could be more than one reference spectrum stored, for different conditions, e.g. different applied used loads (this is discussed further below). In all cases, the reference frequency spectrum has dominant peak corresponding to a main harmonic component, and has a bandwidth associated with the dominant peak, and has a quality factor associated with the dominant peak. By comparing characteristics of a frequency spectrum of a sensed oscillatory motion pattern with characteristics of the reference oscillatory motion pattern, a level of tooth mobility may be detectable.

Alternatively, characteristics of the sensed frequency spectrum could be used alone as the basis for determining a level of tooth mobility, e.g. by monitoring changes therein for the same user over time, or by using a pre-determined transfer function or lookup table.

The sensor means 30 referred to above may take different forms, for example a pressure sensor, force sensor, inertial sensor (e.g. accelerometer), or load cell. The sensor means may be adapted to sense variations of acceleration, pressure, and/or force in the support body or the cleaning elements in one or more directions, e.g. normal to the support body surface from which the cleaning elements protrude, or preferably, in the three orthogonal axes.

The processing arrangement in this set of embodiments is adapted to convert the sensed signal variations into a tooth mobility level or grade identification.

In some embodiments, the processing arrangement may be adapted to compare the one or more characteristics of the sensing signal waveform with corresponding characteristics for a stored baseline or reference sensing signal to determine the tooth mobility level. The reference signal may for example correspond to a signal for a certain reference tooth (e.g. permanent healthy tooth which could be indicated by a dental practitioner).

In some embodiments, an averaged sensing signal for multiple cleaning sessions may be computed by storing the sensing signal for each session in a memory and then retrieving the stored signals and computing the average. This average signal could be used for determining the level of tooth mobility instead of (or in addition to) the signal for just a single cleaning session.

In some embodiments, the measure of tooth mobility could be derived for a given tooth by comparing the one or more characteristics of the signal waveform for the given tooth with those of one or more other teeth acquired within the same cleaning session.

In at least one set of embodiments, it is proposed to determine tooth mobility using a spectral analysis of the sensing signal. For example, determining tooth mobility may comprise assessing, within the frequency domain of the sensing signal, variations of signal amplitude at one or more frequency points, and/or damping of a main harmonic component of the oscillation pattern over one or more cleaning sessions. The variations may be monitored over multiple cleaning sessions, or within the same cleaning session using differential measurement among different teeth.

As will be described further below, the processing arrangement may be further adapted to: process a waveform of the signal to extract one or more characteristics of the signal waveform for the time period, and determine the measure indicative of a degree of tooth mobility in dependence upon the extracted one or more signal waveform characteristics.

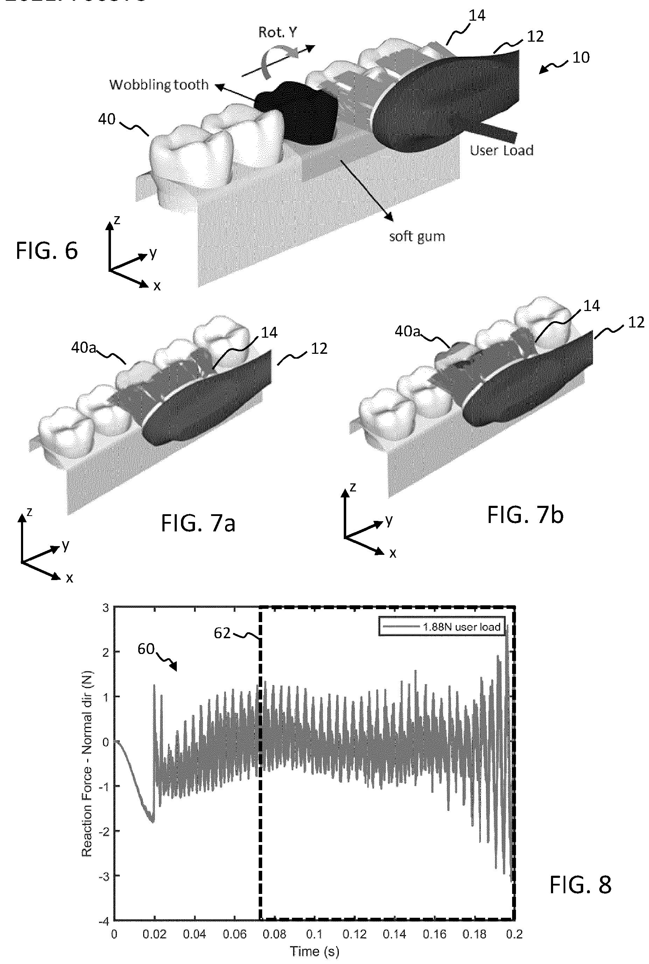

With reference to Fig. 6 and Fig. 7, the sensing principle is illustrated. Fig. 6 shows mechanical engagement of cleaning elements 14 of an example oral care device 10 with teeth 40. The user, in cleaning their teeth, applies the cleaning elements with a certain user load force, which includes a component normal to the surface of the tooth. As illustrated in Fig. 6, when the cleaning elements engage a tooth which has a high level of mobility ("wobbly tooth"), the tooth exhibits motion. The motion of the highlighted tooth 40a is predominantly motion about the y-axis, but also exhibits some smaller degree of motion about the x-axis.

Fig. 6 and Fig. 7 represent computer simulations which were run to test and demonstrate the feasibility of detecting tooth mobility. The model employed an *in-silico* tooth-jaw model.

Fig. 7(a) shows a simulation in which the tooth of interest 40a demonstrates a low level of tooth mobility (the maximum tooth displacement being 0.05mm, representative of normal physiological tooth movement). Fig. 7(b) shows a simulation in which the tooth of interest is a wobbling tooth demonstrating a high level of tooth mobility (the maximum tooth displacement being 1.5mm, a large wobbling amplitude, typical of tooth mobility for a tooth that will subsequently be lost).

The in-silico model consisted of a jaw with soft gums around the reference tooth 40a, and wherein the reference tooth is free to rotate around the mesial-distal (Y) axis and is connected through a torsional spring to the root, as schematically in Fig. 6.

The level of mobility of the reference tooth is controlled in the model by tuning the stiffness coefficient of the torsional spring attached to it. Brushing conditions were simulated based on a Philips A3 brush head, with a 90° angle of the cleaning elements relative to the tooth surface (90° brushing angle) and a constant user load of 1.88N (average load among Philips toothbrush users). The motion pattern of the cleaning elements was modeled as a large-amplitude medial-distal stroke (applied by a user) superposed with a smaller amplitude oscillatory motion pattern induced by the actuation/drive mechanism of the powered toothbrush.

A simulated sensing signal representing reaction forces on the brush head platen 12 as a function of time, as well as the simulated tooth displacement, were computed from the model in the two mobility conditions of Fig. 7(a) and Fig. 7(b).

Fig. 8 shows a representation of a waveform 60 of the obtained simulated sensing signal in the time domain.

The received sensing signal waveform 60 may typically comprise signal segments corresponding to respective periods of engagement of the at least one cleaning element with each of a plurality of teeth. The processing arrangement 20 may be adapted to determine a respective metric of tooth mobility for each of the plurality of teeth. An identification of each of the teeth may further be obtained in some examples.

Within the simulated sensing signal result of Fig. 8, a signal segment 62 corresponding to the modelled tooth of interest 40a is indicated.

In accordance with at least one set of embodiments, determining the degree of tooth mobility may comprise performing a spectral analysis of the sensing signal.

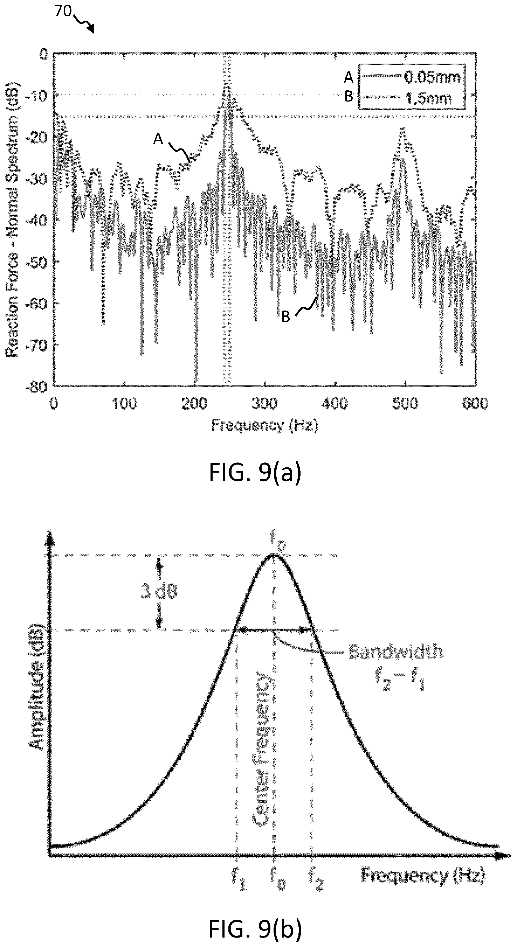

This is illustrated further with reference to Fig. 9(a).

To compute the measure indicative of a degree of tooth mobility, the processing arrangement in accordance with at least one set of embodiments is adapted to compute a frequency spectrum 70 for the sensing signal.

The plot of Fig. 9(a) shows a frequency spectrum for each of the two different tooth mobility levels, labelled A, B, which were illustrated in Fig. 7(a) and (b). In particular, level A corresponds to a mobility amplitude of 0.05 mm. Level B corresponds to a mobility amplitude of 1.5 mm. The illustrated frequency spectrum was computed for the normal reaction force components (x-direction in Figs. 6-7) of the sensing signal at each of the two different tooth mobility levels, A, B.

As mentioned above, in this, the oral care device is one which includes an actuation means 19 for driving an oscillatory motion of the at least one cleaning element 14 which, exhibits a baseline/reference frequency spectrum with a dominant peak having a central frequency component around the driving frequency.

Observing the frequency domain plot 70 for the reaction forces, there is a large increase in the reaction force amplitude levels for frequency components around the harmonics of the dominant frequency (around 250 Hz) of the reference frequency spectrum of the actuation means. There is also an increase in the damping level between frequency components around these harmonic frequencies. In particular, the amplitude of the frequency component corresponding to the dominant frequency component (i.e. the main harmonic component) of the oscillatory driving force applied by the actuation means (at around 250 Hz) shows an amplitude increase of about 5.4dB (in the normal direction) between the lowest and the highest level of tooth mobility simulated.

The changes in the damping around the dominant actuation frequency harmonics are qualitatively observable by the changes in bandwidths, and could be determined quantitatively by, for instance, computing the quality factor (Q factor), i.e. a dimensionless parameter defined as the ratio of the resonance center frequency to its bandwidth.

This is illustrated schematically in Fig. 9(b), which shows how the Q-factor can be computed as the ratio of the central frequency, f₀, to the bandwidth (f₂-f₁), i.e. Q-factor = f₀/ (f₂-f₁).

For example, for a -3dB bandwidth, the Q factor for the main harmonic component changes from 36.5 in the case of low level of mobility case A (max 0.05mm displacement) to 34.4 for the high level mobility case B (max 1.5mm displacement).

Thus, following this approach, in accordance with at least one set of embodiments, determining the degree of tooth mobility may comprise detecting an amplitude of a central frequency of a dominant peak in the frequency spectrum of the sensing signal (i.e. the central frequency of the main harmonic peak or main resonant peak). In particular, the processing arrangement may detect changes in the amplitude of this central frequency of the dominant peak and/or in damping over multiple brushing sessions in one tooth or multiple teeth. The assessment can also be made in a single brushing session by using the spectral signature associated to a non-wobbling/permanent tooth as reference.

In some examples, determining the degree of tooth mobility may be performed by determining changes between the reference frequency spectrum of the oscillation applied by the actuation means and the sensed frequency spectrum of an amplitude of the central frequency component of the dominant peak in the oscillation frequency spectrum .

Additionally or alternatively, in some examples, determining the degree of tooth mobility may be performed by determining changes between the frequency spectrum of the oscillation applied by the actuation means and the sensed frequency spectrum of: a bandwidth associated with a dominant peak in the frequency spectrum, or a quality factor associated with dominant peak of the frequency spectrum.

Thus, in general, the detection of tooth mobility is based on assessing whether the variation in one or multiple characteristic parameters (amplitude and/or damping level of main harmonic component of the frequency spectrum of the force, pressure or acceleration measured signal) surpasses a threshold value.

For example, if ΔA = A₂- A₁> θ_{A} and/or Δd = d₂- d₁> θ_{d}, then considerably large tooth mobility is detected, with Aᵢ being the amplitude of the main harmonic component of measured signal, dᵢ being the associated damping measure at state i, and θ_{A}, θ_{d} being respective thresholds.

For example, the damping measure could be given by 1/Q_{-3dB} with Q_{-3dB} being the Q factor for -3dB bandwidth. The example reference bandwidth -3dB is exemplary only and another reference bandwidth could instead be used. Any other way of assessing damping from the frequency spectrum plot could be used, for instance modal curve fitting.

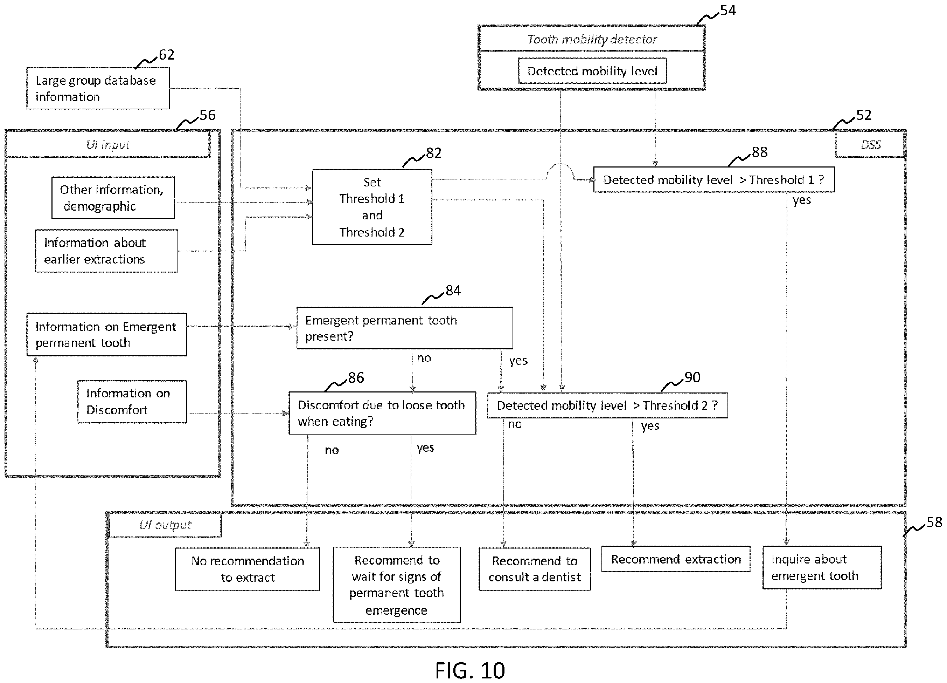

A detailed implementation of a method according to one or more embodiments will now be outlined, with reference to Fig. 10.

In a preferred embodiment, a simple rule-based Decision Support System (DSS) module is provided by the programming of the processing arrangement, and is adapted to triage a given situation towards a recommended action, if any. The DSS is for example an algorithm executed by the processing arrangement.

Fig. 10 schematically illustrates an example processing flow of the processing arrangement.

The processing arrangement includes a DSS software module 52 which processes various data inputs to provide one or more data outputs.

Fig. 10 schematically shows a degree of tooth mobility of at least one tooth being provided as one input, from a tooth mobility data source 54. This could be a software module of the processing arrangement which processes input sensor data to obtain the tooth mobility measure. In other cases, it could be manually input for example.

Fig. 10 further illustrates as another input an indicator of the emergence level of the permanent tooth. In Fig. 10, this is shows as being provided as in input from a user via the user interface input device 56. However, in further example, it could be computed automatically, in accordance with the methods described in detail above.

Fig. 10 further illustrates as another input an indicator of a discomfort level of the child. This can be input using the user interface input device 56.

Fig. 10 further illustrates as another input child-specific information, such as demographic information relating to the child (demographic profile). This can be input using the user interface input device 56.

Fig. 10 further illustrates as another input information about earlier extractions performed on the same child. This can be input using the user interface input device 56.

Fig. 10 further illustrates that another input to the DSS module 52 could be information sourced from a database 62 containing information related to natural tooth release for a large group of children. For example, the DSS 52 could perform a comparison of one or more of the information sources mentioned above relating to the child and the teeth to the data in the population database 62, and wherein the recommendation(s) generated by the DSS are based in part on this comparison.

Additionally or alternatively, the DSS may comprise a machine-learned algorithm which has been trained in advance to generate recommendation(s) based on the above-mentioned input data, and wherein the training utilized the data in the database 62 as the training data.

As shown in Fig. 10, the child-specific information might be used to set a first and second threshold 82 for the tooth mobility, to be used in assessing tooth mobility. These represent respective criteria to be used in determining what recommendation to provide. The second threshold (threshold 2) represents a threshold mobility for safe extraction (i.e. with no or negligible bleeding), where this can be based on the experience with past teeth of the same child or from population/expert knowledge. The first threshold is a threshold mobility for checking for signs of permanent tooth emergence.

The detected mobility level is compared 88 with the first threshold. If the mobility is greater than the first threshold then a prompt is provided via the user interface recommending that the parent check for permanent tooth emergence at the site of the given tooth. It may prompt the user to provide a user input indicative of whether there are signs of permanent tooth emergence. This information is then used as an input to other parts of the DSS processing.

In particular, the user-input information relating to permanent tooth emergence is used to determine an indicator of a permanent tooth emergence status 84.

If the permanent tooth emergence indicator 84 is negative, then the input information on discomfort of the child is used to determine what output recommendation to provide. For example, if there is no discomfort when eating, then no recommendation to extract is provided. If there is discomfort when eating or brushing, then a recommendation may be issued to wait for first signs of permanent tooth emergence before considering extracting. This recommendation could also include an indication that extraction would nonetheless be safe at this point, if needed.

If the determined permanent tooth emergence status 84 is positive, then the tooth mobility level may be compared 90 with the second threshold (higher than the first). If the tooth mobility level exceeds the second threshold, then a recommendation may be issued to extract the tooth. If the mobility level does not exceed the second threshold, then a recommendation may be issues to consult a dental practitioner.

The various recommendations may be provided via a user output component 58 of the user interface.

In some embodiments, the system may execute a calibration phase based on at least one tooth of the child which is extracted, and defines the criteria for use in generating recommendations based on input feedback related to the experience with the first tooth. For example, if there was no or little bleeding, the system saves the latest measured mobility level as a safe level for extraction. In some embodiments, the DSS algorithm may learn from population data via reinforcement learning wherein the reinforcement reward includes the resulting bleeding, the discomfort up to the tooth loss and the potential misplacement of the emergent permanent tooth.

Embodiments of the invention described above employ a processing arrangement. The processing arrangement may in general comprise a single processor or a plurality of processors. It may be located in a single containing device, structure or unit, or it may be distributed between a plurality of different devices, structures or units. Reference therefore to the processing arrangement being adapted or configured to perform a particular step or task may correspond to that step or task being performed by any one or more of a plurality of processing components, either alone or in combination. The skilled person will understand how such a distributed processing arrangement can be implemented. The processing arrangement includes a communication module or input/output for receiving data and outputting data to further components.

The one or more processors of the processing arrangement can be implemented in numerous ways, with software and/or hardware, to perform the various functions required. A processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A processing arrangement (20) for an oral care device, comprising:
an input/output (22); and
one or more processors (24), adapted to:
obtain an indication of a degree of tooth mobility of at least one infant tooth in the mouth of a user of the oral care device;
assess the degree of tooth mobility according to at least one pre-defined criterion for the tooth mobility; and
responsive to the degree of tooth mobility meeting the at least one pre-defined criterion, either:
generating an output signal at the input/output for causing a user interface element to generate a prompt recommending that a user manually check for emergence of a permanent tooth at the site of the at least one infant tooth; or
generating a control signal at the input/output for controlling acquisition of an indicator of permanent tooth emergence status based on sensing or image data for the at least one infant tooth.

2. The processing arrangement of claim 1, further adapted to receive at the input/output sensing data related to an oral cleaning action performed using the device during an oral cleaning session, and wherein the degree of tooth mobility is determined based on the sensing data.

3. The processing arrangement of claim 1 or 2, further adapted to:
receive at the input/output (22) a sensing signal (32) indicative of a loading force or pressure applied to, or motion of, at least one cleaning element (14) of the oral care device, wherein the signal spans a time period, the time period corresponding to a period of mechanical engagement of the at least one cleaning element with at least one tooth (40) during an oral cleaning session, wherein the tooth is the at least one infant tooth;
determine the measure indicative of a degree of tooth mobility for at least one tooth based on processing of the sensing signal.

4. The processing arrangement of claim 3, further adapted to: process a waveform (60) of the signal to extract one or more characteristics of the signal waveform for the time period, and determine the measure indicative of a degree of tooth mobility in dependence upon the extracted one or more signal waveform characteristics.

5. The processing arrangement of any of claims 1-4, further adapted to obtain an indicator of a permanent tooth emergence status at the site of the at least one infant tooth.

6. The processing arrangement of claim 5, wherein the indicator of a permanent tooth emergence status comprises:
a binary parameter indicative of emergence or non-emergence, or
a graded score indicative of a level of emergence.

7. The processing arrangement of claim 5 or 6, wherein indicator of a permanent tooth emergence status is obtained:
by processing the obtained degree of tooth mobility with a pre-determined tooth emergence algorithm;
based on image data of the site of the at least one infant tooth, received at the input/output from an image capture device, and based on processing of the image data with a predetermined image analysis algorithm; and/or
based on receiving at the input/output a user input signal from a user interface element indicative of the permanent tooth emergence status.

8. The processing arrangement of any of claims 5-7, wherein the one or more processors are adapted to:
access a datastore recording historical tooth mobility data for the at least one tooth of the user;
determine a longitudinal trend in the degree of tooth mobility for at least one tooth of the user based on the historical tooth mobility data and the determined degree of tooth mobility; and
wherein the indicator of a permanent tooth emergence status is determined in dependence on the longitudinal trend.

9. The processing arrangement of any of claims 5-8, further adapted to:
generate an infant tooth extraction recommendation in dependence upon the degree of tooth mobility and the indicator of permanent tooth emergence status; and
generate a data output indicative of the recommendation.

10. The processing arrangement of claim 9, wherein
the infant tooth extraction recommendation comprises a binary recommendation indicating whether or not the at least one tooth should be extracted.

11. The processing arrangement of claim 9 or 10, wherein
determining the tooth extraction recommendation is further based on a demographic classification of the user; and/or
the processing arrangement is further adapted to receive from a user interface an input indicative of a subjective discomfort level of the user, and wherein the determining the tooth extraction recommendation is further based thereon.

12. The processing arrangement of any of claims 9-11, wherein the recommendation is a recommendation to extract the tooth in the case that both:
the permanent tooth emergence status is positive; and
the mobility exceeds a predetermined extraction threshold.

13. An oral care device comprising:
a support body (12);
a plurality of cleaning elements (14) for engagement with oral surfaces (42), the cleaning elements protruding from a surface of the support body; and
a processing arrangement (20) in accordance with any of claims 1-12.

14. The device of claim 13, further comprising:
a sensor means (30) adapted to sense a measure indicative of a loading force or pressure applied to, or motion of, at least one of the cleaning elements (14).

15. A computer implemented method comprising:
obtaining an indication of a degree of tooth mobility of at least one infant tooth in the mouth of a user of an oral care device;
assessing the degree of tooth mobility with at least one pre-defined criterion for the mobility; and
responsive to the degree of tooth mobility meeting the at least one pre-defined criterion, either
generating an output signal for causing a user interface element to generate a prompt recommending that a user manually check for emergence of a permanent tooth at the site of the at least one infant tooth; or
generating a control signal for controlling acquisition of an indicator of permanent tooth emergence status based on sensing or image data for the at least one infant tooth.
